# EUROPEAN PATENT APPLICATION

(11) **EP 2 284 541 A1**
(43) Date of publication of application: **16.02.2011**
(21) Application number: 09010408.4
(22) Date of filing: 12.08.2009
(51) Int. Cl.: G01N 33/92, G01N 1/30

(54) **Fluorescence-based imaging and analysis of cells and cellular components using lipophilic dyes with improved specificity, spectral property and photostability**

(71) Applicant: Max-Planck-Gesellschaft zur Förderung der Wissenschaften e.V., 80539 München (DE)
(72) Inventor: Thiele, Christoph, 01328 Dresden (DE)
(74) Representative: Katzameyer, Michael

(57) **Abstract**

The present invention relates to the use of specific bodipy dyes having the general structural formula I below 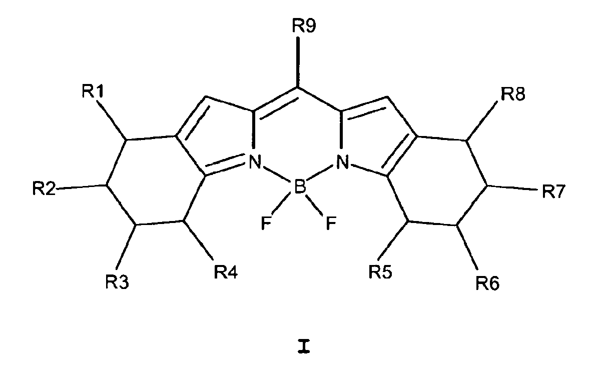 wherein the substituents R₁, R₂, R₃, R₄, R₅, R₆, R₇, R₈ and R₉ independently are H or an organic residue, for fluorescence-based imaging, in particular multi-color imaging, or analysis of cells and cellular components including lipid droplets.

In a preferred embodiment, R₁, R₂, R₃, R₄, R₅, R₆, R₇, R₈ and R₉ in formula I above are independently H or an unsubstituted or substituted lower alkyl group having 1-8 C atoms, such as methyl, ethyl, propyl, butyl etc. In a further preferred embodiment the substituents R₁, R₂, R₃, R₄, R₅, R₆, R₇, R₈ in formula I are each H and R₉ is an alkyl group, preferably an lower alkyl group having 1-8 C atoms.

The present invention can be advantageously applied to the fields of microscopy, flow cytometry or fluorescence activated cell sorting (FACS).

## Description

### Background of the Invention

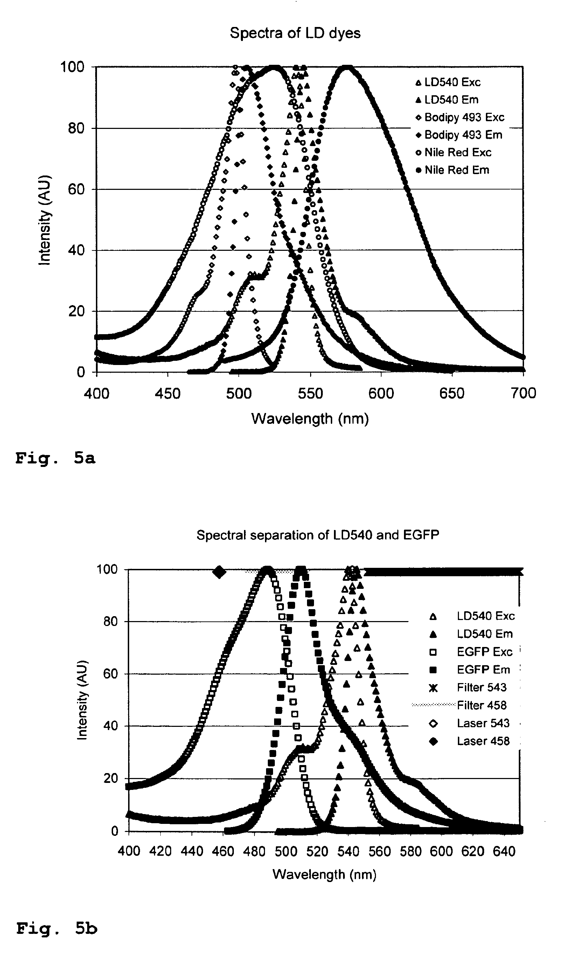

Lipid droplets (LDs) are the cellular organelles that store triglyceride and other neutral lipids. They consist of a core of neutral lipid, surrounded by a monolayer of phospholipids with attached or embedded proteins. LDs are dynamic organelles, which are formed and degraded, move inside cells and also may undergo fusion. To study their morphological and dynamic relation to other cellular organelles, multi-color fluorescence imaging of fixed specimens or living cells is an essential tool. Since LDs form functional and morphological subpopulations (Wolins et al., J. Biol. Chem. 2005; 280(19):19146-19155), no single marker protein exists which would stain the entire LD pool of a cell. Therefore, the only reliable way to visualize all droplets in a given cell is by staining the LD core with lipophilic dyes. So far, mainly two dyes, Nile Red and Bodipy 493/503, have been used for this purpose (Greenspan et al., J. Cell. Biol. 1985; 100(3):965-973; Gocze and Freeman, Cytometry 1994; 17(2):151-158). Nile Red displays strong solvatochromism and broad excitation and emission bands, being visible using filter sets for both green and red dyes (see Fig. 5a for spectra of the LD dyes). Nonetheless, Nile Red can be imaged together with EGFP for applications that do not require complete separation of channels (Miura et al., J. Biol. Chem. 2002; 277(35):32253-32257), but addition of other yellow, orange or far-red dyes is not possible. Bodipy 493/503 can be spectrally separated from many red fluorescent dyes and proteins, but not from the popular fluorescent proteins ECFP, EGFP, EYFP or fluorophores with similar properties, precluding multi-color imaging in living cells. A recent addition are two red fluorescent LD dyes, LipidTox red and LipidTox far red, that can be spectrally separated from green fluorophores and have occasionally been used for LD staining (Granneman et al., J. Biol. Chem. 2009; 284(5):3049-3057). Judged on the pictures provided by the manufacturer (Invitrogen. HCS LipidTOX™ Neutral Lipid Stains. 2006 [available from: probes.-invitrogen.com/media/-pis/mp34475.pdf 6), there seem to be considerable problems with background staining. There is no publication that describes the dyes' properties, and the manufacturer does not disclose their chemical identity and sells them currently for about 200-fold the price of Bodipy 493/503.

In view of the drawbacks of the lipophilic fluorescent dyes generally used for imaging, in particular multi-color imaging, of cells and cellular components and in particular for LD staining in the prior art, a main object of the present invention was to provide a generally applicable solution to the problem of imaging, in particular multi-color imaging, of cellular components, including LDs, by the use of fluorescent dyes which exhibit improved properties, such as improved specificity, brightness and photostability, and which can spectrally be resolved from other fluorophores, in particular both green and red fluorophores, allowing multi-color imaging in both fixed and living cells. A related object was to provide improved means for fluorescence-based analysis of cells and cellular components, in particular lipophilic components.

These objects have been achieved by providing the novel use of specific bodipy dyes according to claims 1-7 and 15, as well as by providing the methods of claims 8-14.

### Description of the Invention

The specific bodipy dyes which are advantageously used in the present invention are represented by the general structural formula **I** below 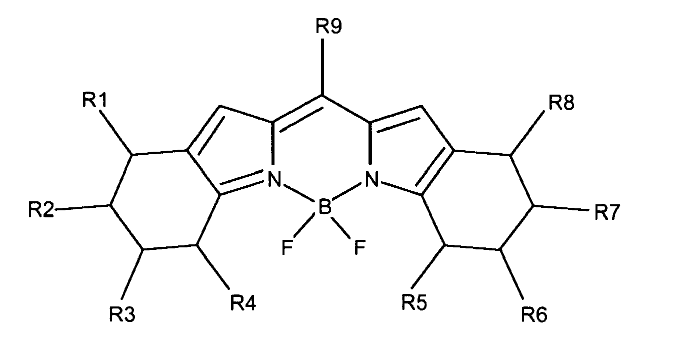 wherein the substituents R₁, R₂, R₃, R₄, R₅, R₆, R₇, R₈ and R₉ independently are H or an organic residue. The organic residue may be e.g. an unsubstituted or substituted (cyclo)alkyl group, alkenyl group, alkinyl group, heterocyloalkyl group, aryl group, heteroaryl group, a functional group comprising at least one heteroatom such as N, O, S, P or halogen, or any combination of said groups.

The term "aryl", as used herein above, refers to a mono-, bi- or tricyclic carbocyclic ring system having one, two or three aromatic rings including but not limited to phenyl, naphthyl, anthryl, azulyl, tetrahydronaphthyl, indanyl and indenyl.

The term "heteroaryl", as used herein above, refers to a cyclic aromatic radical having from 5 to 10 ring atoms of which at least one ring atom is selected from S, O and N; the radical being joined to the rest of the molecule via any of the ring atoms. Representative, but not limiting examples are pyridinyl, pyrazinyl, pyrimidinyl, pyrrolyl, imidazolyl, thiazolyl, oxazolyl, isooxazolyl, thiadiazolyl, oxadiazolyl, thiophenyl, furanyl, quinolinyl and isoquinolinyl.

The term "heterocycloalkyl" as used herein above, refers to a non-aromatic 3-, 4-, 5-, 6- or 7-membered ring or a bi- or tricyclic group comprising fused 6-membered rings having between 1 and 3 heteroatoms independently selected from S, O and N, including but not limited to pyrrolidinyl, pyrazolinyl, pyrazolidinyl, imidazolidinyl, piperidinyl, piperazinyl, oxazolidinyl, isoxazolidinyl, isooxazolidinyl, morpholinyl, thiazolidinyl, isothiazolidinyl, and tetrahydrofuryl.

The "functional group comprising at least one heteroatom such as N, O, S, P or halogen", may for example comprise OH, OR, in particular alkoxy, COOH, COOR, an unsubstituted or substituted amine group, NO₂, SO, SO₂, SO₃H, SO₃R, F, Cl, Br, I, but is not limited thereto.

The term "alkyl group", as used herein, comprises a substituted or unsubstituted alkyl group, including a cycloalkyl group. The alkyl group(s) may be branched or unbranched and preferably have 1-30, more preferred 1-20, still more preferred 1-10, C atoms. Especially preferably, the alkyl group(s) comprise an unsubstituted or substituted lower alkyl group having 1-8 C atoms, preferably 1-6 or 1-4 C atoms, such as methyl, ethyl, propyl, butyl etc.

In a preferred embodiment, R₁, R₂, R₃, R₄, R₅, R₆, R₇, R₈ and R₉ in formula I above are independently H or an unsubstituted or substituted lower alkyl group having 1-8 C atoms, preferably 1-6 or 1-4 C atoms, such as methyl, ethyl, propyl, butyl etc.

In a further preferred embodiment the substituents R₁, R₂, R₃, R₄, R₅, R₆, R₇, R₈ in formula **I** are each H and R₉ is an alkyl group, preferably an unsubstituted or substituted lower alkyl group having 1-8 C atoms, such as methyl, ethyl, propyl, butyl etc. Most preferred, R₉ is methyl (formula **II** below).

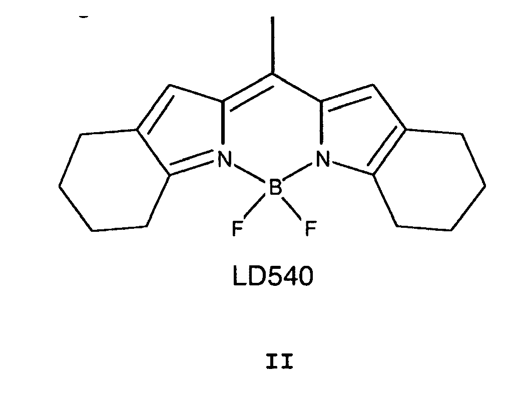

This dye is denominated 4,4-Difluoro-2.3,5.6-bis-tetramethylene-4-bora-3a,4a-diaza-s-indacene according to IUPAC nomenclature and will be briefly referred to as LD540 in the present application. The same compound has been described as compound 14 in the review by Loudet and Burgess (BODIPY dyes and their derivatives: syntheses and spectroscopic properties. Chem. Rev. 2007; 107(11):4891-4932), and was originally published as compound 7h in the patent by Morgan and Boyer (U.S. Patent No. 5,446,157), with reported excitation and emission maxima of 535 nm and 560 nm, respectively. However, this compound has been disclosed by Morgan and Boyer merely as one specific member of a large generic group of bodipy dyes reported to be useful for dye laser systems and photochemical agents in the treatment of diseased tissues using photodynamic therapy techniques. The use of said compound in staining and (multi-color) imaging of cells or cellular components, in particular lipid droplets, has neither been disclosed nor suggested in this prior art.

LD540 has a number of favorable characteristics which render this compound and structurally related compounds especially suited for use in multi-color imaging and staining of cellular components. This lipophilic dye is (i) red-shifted relative to Bodipy 493/503 which allows spectral separation from GFP, (ii) is lipophilic enough to partition into LDs, (iii) has a symmetric structure that can be built in a one-pot reaction from (iv) commercially available precursors to allow economic synthesis.

The structure of LD540 and related compounds differs from the commonly used dye Bodipy 493/503 mainly in the presence of the two 6-membered cycloalkyl rings which flank the tricyclic core system and are largely responsible for the favorable spectral characteristics of these compounds. In particular compounds wherein R₁, R₂, R₃, R₄, R₅, R₆, R₇, R₈ and R₉ in formula **I** above are independently H or an alkyl group, in particular an unsubstituted or substituted lower alkyl group having 1-8 C atoms, preferably 1-6 or 1-4 C atoms, such as methyl, ethyl, propyl, butyl etc, have spectral characteristics which are very similar to those of LD540. The skilled artisan will be readily able to modify the above basic structure of LD540 by varying the substituents R₁-R₉ by methods known in the art and conducting routine trials to identify further suitable compounds with similar favorable spectral characteristics as those of LD540.

In a closely related aspect, the present invention also provides improved methods for imaging, in particular multi-color imaging, of cells and/or (lipophilic) cellular components, in particular lipid droplets.

In a specific embodiment of this aspect, the inventive method comprises the following steps:
a) staining cellular components with a bodipy dye of formula **I** as defined in any one of claims 1-4;
b) staining other cellular components with at least one other excitable fluorescent dye having a different maximum of the excitation and/or emission spectrum than the bodipy dye used in step a);
c) exciting the bodipy dye of step a) with laser light in the wave length range from about 450 to about 565 nm, preferably about 488 nm to 561 nm, more preferred 488 to 543 nm;
d) exciting the at least one other excitable fluorescent dye of step b) with laser light in a wave length range different from that used to excite the bodipy dye of formula I;
e) detecting the light emitted by the different dyes used in step a) and step b);
f) optionally generating images with the emission data obtained in step e);
g) optionally performing an analysis with the data obtained in step e) or the images obtained in step f).

The cellular components stained with the bodipy dye of formula I in step a) may be any lipophilic cellular components and are preferably lipid droplets.

The at least one other excitable fluorescent dye used in step b) may be any excitable fluorescent dye known in the art and in particular any fluorescent dye known to be suited for selective staining and imaging of cellular components. Suitable dyes may be selected from the group comprising Cy dyes, Alexa dyes such as Alexa 647, NBD, fluorescein and its derivatives such as cascade blue and Oregon green, fluorescent proteins such as EGFP, emerald, EYFP, mRFP, morange, mPlum, mcherry, DAPI, and Texas Red, but are not limited thereto.

The maxima of the excitation and/or emission spectra of these dyes have to be sufficiently separated from each other and in particular from the corresponding maximum of the bodipy dye of formula **I** to allow resolving the emission of the different dyes from each other. Suitable dyes can be selected by the skilled artisan on the basis of their known spectral characteristics and/or routine trials. Preferably, the maxima of the excitation and/or emission spectra of the bodipy dye of formula **I** will be separated from the corresponding maxima of said one or more other excitable fluorescent by a value of at least 25 nm, in order to enable or facilitate resolving the emission of the bodipy dye of formula I from the emission of said one or more other excitable fluorescent dyes.

In specific embodiments of the method according to the present invention, 1, 2 or 3 other excitable fluorescent dye(s) are used in step b), resulting in 2-color, 3-color or 4-color imaging.

The wave length of the laser light used for exciting the bodipye dye in step c) will depend from the spectral characteristics the specific dye used and the intended specific purpose and can be assessed and optimized by the skilled artisan in routine trials. For LD 540, preferred wave lengths are 488 nm, 514 nm, 532 nm or 543 nm.

A very favourable feature of the methods of the present invention resides in the fact that not only fixed cells but also living cells can be subjected to said imaging, in particular multi-color imaging, of cells and/or cellular components.

The uses and methods of the present invention in particular may be advantageously applied to the fields of microscopy, flow cytometry or fluorescence activated cell sorting (FACS).

Due to the favourable spectral characteristics of the specific bodipy dyes of formula I the uses and methods of the present invention are especially suited for imaging, in particular multi-color-imaging, in various microscopic applications, including sophisticated applications such as time resolved studies and/or high resolution microscopy such as STED. However, the improved properties of these dyes, particularly their better specificity and stability, will also considerably improve the signals in flow cytometry and FACS. The latter are important applications in medical diagnosis and research and have a significant economic potential.

Typically, in the inventive microsopic methods a confocal laser scanning microscope is used but these methods are applicable to other types of instruments such as a spinning disc microscope, a two-photon microscope, a conventional epifluorescence microscope, a structured illumination microscope, or a single plane illumination microscope as well.

### General Synthesis and spectral properties of LD540

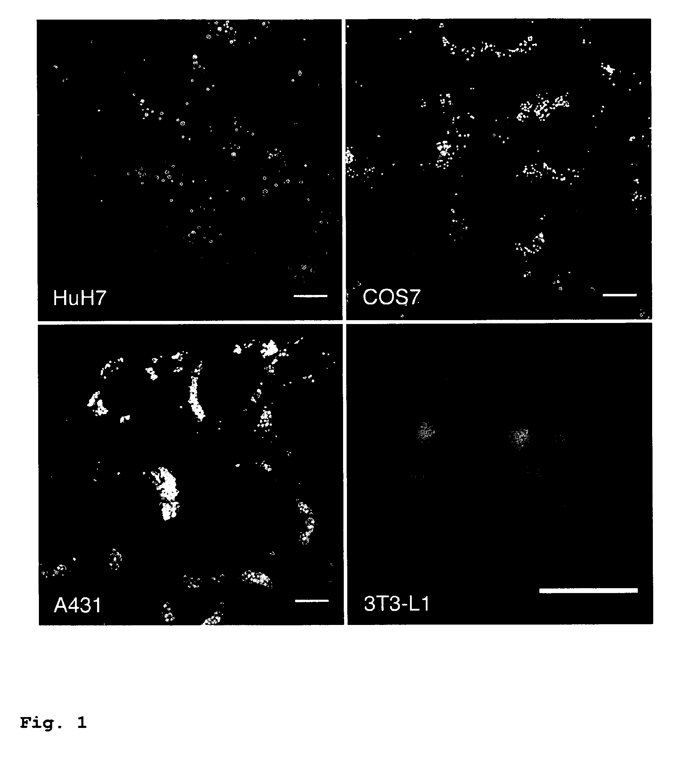

LD540 was synthesized from tetrahydropyrrole, acetylchloride and BF₃-etherate by a method known per se (see Example 1 for details) and various cell lines were stained according to the commonly used protocols for Bodipy 493/503 or Nile Red (Listenberger and Brown; Curr. Protoc. Cell Biol. 2007; Chapter 24:Unit 24 22) (see Example 6 for details). At the concentrations typically used for Bodipy 493/50, staining by LD540 was much brighter. Reducing the dye concentration resulted in a bright, robust and highly specific yellow-green staining of LDs in all cell lines (Fig. 1). Since the absorption coefficient and the quantum yield of LD540 are similar to that of Bodipy 493/503 (compare U.S. Patent No. 5,446,157), brighter staining and increased specificity indicate stronger accumulation of the dye in the LDs.

The present inventors measured log P values of both dyes for partitioning in different solvent pairs and found a significantly higher lipophilicity of LD540 (Example 2, Table 1). This allows use of LD540 in concentrations 10-20 times lower than Bodipy 493/503, reducing costs and background staining considerably.

For use in imaging, in particular multi-color imaging, subsequently its spectral properties were studied under various conditions (Example 3, Table 2). Solutions in different solvents were compared with a suspension of LD540-stained 3T3-L1 cells. Excitation maxima showed a small red-shift with increasing lipophilicity of the solvent. Maxima from stained cells (lₘₐₓ ex = 540 nm, lₘₐₓ em = 545 nm) were identical to those of the dye dissolved in sunflower oil, consistent with the presence of the dye in the intracellular oil droplets.

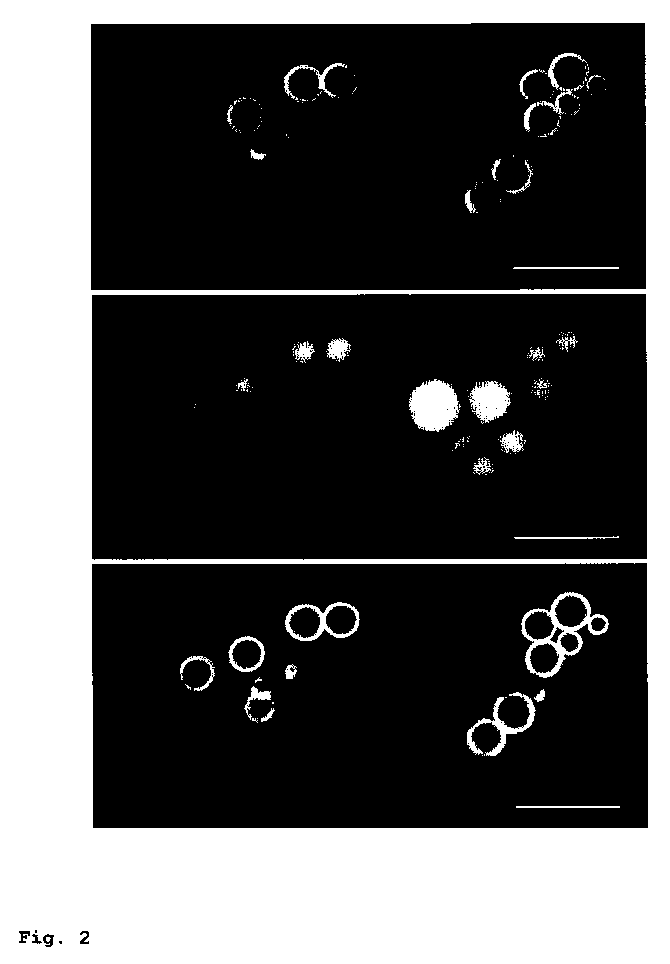

For further analysis (Example 4), therefore, the spectra measured in oil (Fig. 5a) were used since they optimally represent the dye's behavior in cells. Overlay with spectra of the various fluorescent proteins indicated the possibility of spectral resolution from the most popular fluorescent protein, EGFP (Fig. 5b). With these optimized settings, bright, well-defined images could be achieved in living cells (Fig. 2), suitable for image analysis without the need of correction for excitation cross talk or emission bleed through.

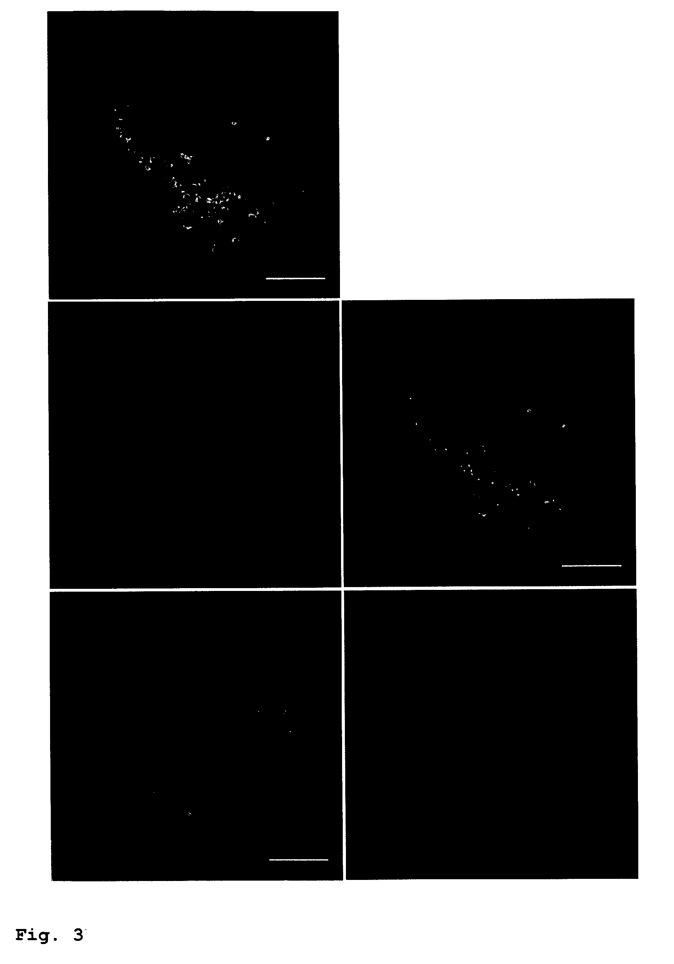

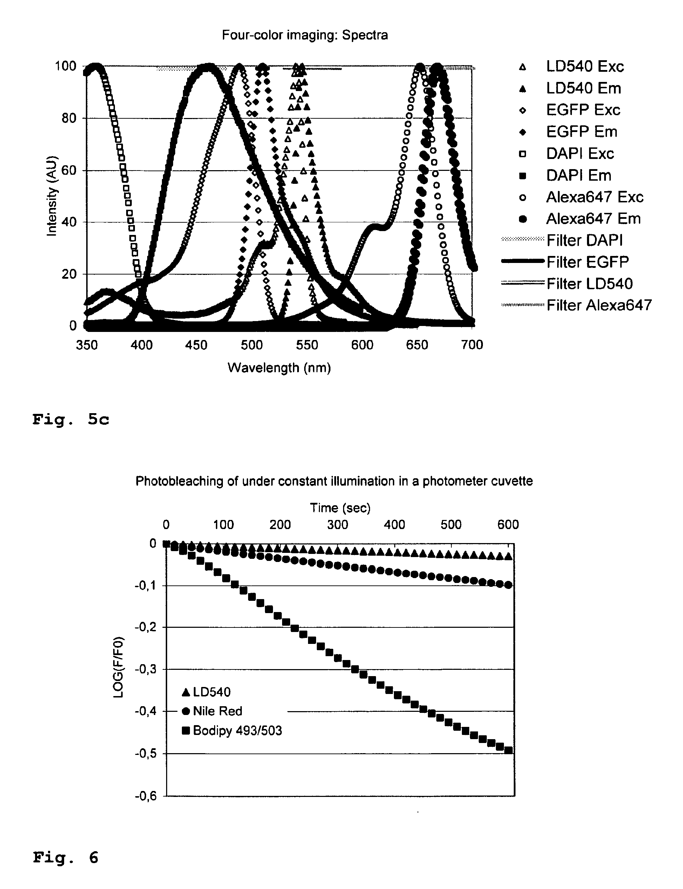

Further addition of blue and far-red fluorophores (see Fig. 5c for spectra) allowed straightforward multi-color imaging. With appropriate laser lines and emission filters, separation of four colors is possible without the need of spectral imaging and mathematical linear un-mixing of the four dyes. Using these settings, multi-color imaging in HuH7 hepatoma cells (Fig. 3) for nuclei (DAPI, blue), a LD-associated protein (Rab18-EGFP, green), the LD core (LD540, red) and filamentous actin (Alexa647-phalloidin, magenta) could be performed, which demonstrated the lack of co-localization of LDs with filamentous actin structures.

Apart from specificity and spectral properties, photostability is the third key feature of a neutral lipid dye, and it is of particular practical importance for imaging. It is limiting the usefulness of a dye for repeated high resolution imaging needed for both time-resolved studies on living cells and modern high-resolution microscopy methods such as structured illumination or STED microscopy (Stemmer et al., Histochem. Cell Biol. 2008; 130(5):807-817). Since Bodipy 493/503 is rapidly bleached (Listenberger and Brown; Curr. Protoc. Cell Biol. 2007; Chapter 24:Unit 24 29), previous attempts of live cell imaging of LDs have either employed GFP-labeled LDs (Pol et al., Mol. Biol. Cell 2004; 15(1):99-110; Turro et al., Traffic 2006; 7(9):1254-1269) or used Nile Red, the latter resulting in background problems particularly when smaller droplets were imaged in non-adipocytes (Pol et al., Mol. Biol. Cell 2004; 15(1):99-110).

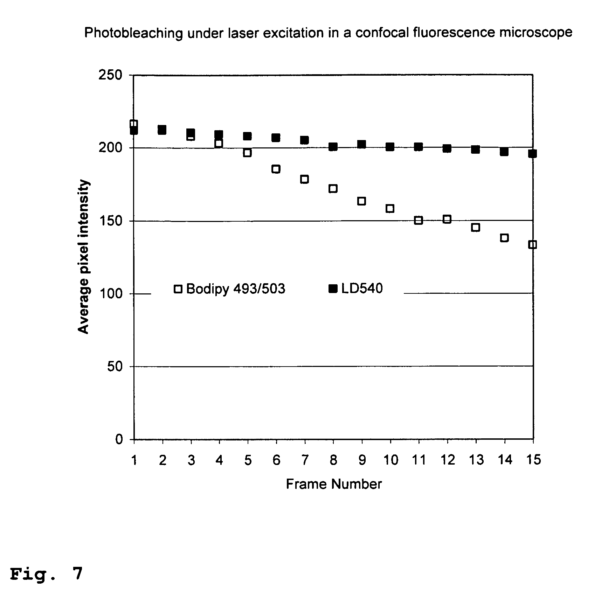

Photobleaching studies under different conditions were conducted by the present inventors (Example 5) and it was found that bleaching of LD540 under constant illumination in a fluorometer was about 15-fold slower than that of Bodipy 493/503, and 3-fold slower than that of Nile Red (Fig. 6). Under laser excitation at conditions practically used for imaging, LD540 was at least four times more stable than Bodipy 493/503 (Fig. 7).

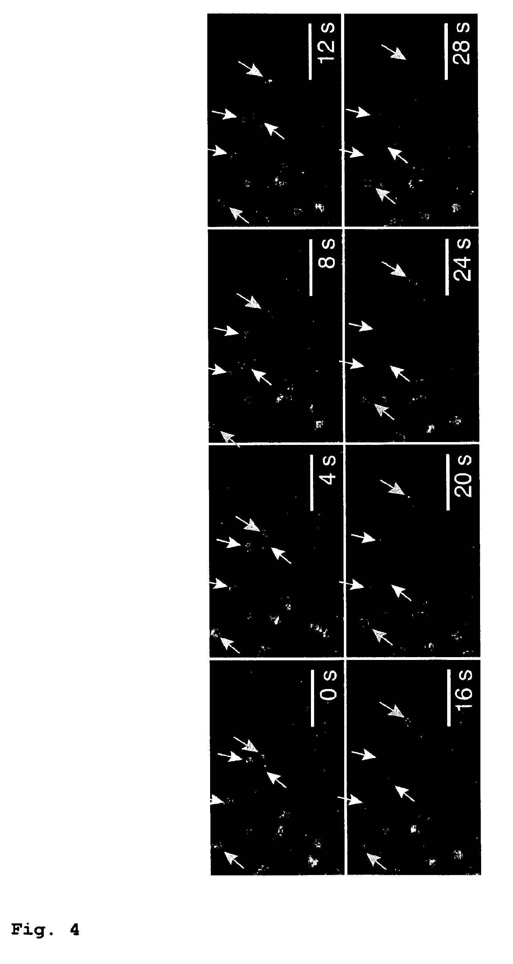

This stability prompted the present inventors to perform two-color live cell imaging of LDs, in order to visualize LD movement along microtubule tracks. So far, motility of LDs along microtubules has been demonstrated indirectly by genetic and biophysical analysis in drosophila embryos. The association of LDs with microtubules has been shown in fixed cells. The present inventors transfected COS7 cells with pEGFP-a-tubulin (Clontech) and stained LDs with LD540. The result is illustrated in Fig. 4, showing a series of eight sequential pictures taken at intervals of 4 s which is part of a movie sequence generated by the inventors (not shown). While a major fraction of LDs (magenta) oscillates around apparent microtubule attachment sites, some LDs show clear processive motility along the microtubule tracks (green). A detailed study of kinetic parameters is beyond the scope of this study, but preliminary analysis of processive LD movements using MotionTracker software (Kalaimoscope, Dresden, Germany) revealed typical velocities of 0.2 - 0.4 µm/s with peak velocities of about 0.7 µm/s, consistent with the reported velocities of LDs in drosophila embryos (Shubeita et al., Cell 2008; 135(6):1098-1107) and in general with kinesin family motors (Hirokawa and Takemura, Exp. Cell Res. 2004;301(1):50-59).

In conclusion, the present invention provides a new tool that allows imaging, in particular multi-color imaging, of LDs in live cells with high specificity, which will be helpful to explore the intracellular transport of neutral lipid and changes in the morphological organization of these fascinating organelles.

The invention is further illustrated by the following nonlimiting Examples and Figures.

### FIGURES

**Fig. 1****.** Different formaldehyde-fixed cells stained with a solution of 50 ng/ml LD540 in PBS for 10 min (laser excitation at 543 nm). Bar: 10 µm.
**Fig. 2****.** Two color-imaging of 3T3-L1 cells transiently expressing AUP1-EGFP and stained with LD540. An overlay of green (AUP1-EGFP) and red (LDs) channels is shown together with the single unprocessed tiff files demonstrating the complete separation of the two channels.
**Fig. 3****.** Multi-color imaging of LDs in HuH7 hepatoma cells. HuH7 cells transiently expressing Rab18-EGFP (green) were fixed, permeabilized, and stained with Alexa647-phalloidin (magenta) for actin filaments, LD540 (red) for LDs and DAPI (blue) for DNA. Bar: 10 µm.
**Fig. 4****.** COS7 cells transiently expressing tubulin-EGFP were stained with LD540 and images taken on the Zeiss LSM510 META every 4 seconds. The images show LDs (magenta) and microtubules (green). Bar, 5 µm.
**Fig. 5****.** **5a**. Excitation and emission spectra of 3 different LD dyes (LD540, Bodipy 493/503, Nile Red) dissolved at 0.1 µg/ml in sunflower oil
**Fig. 5b****.** Overlay of the LD540 spectra from Fig. 5a with the fluorescence spectra of EGFP
**Fig. 5c****.** Four-color imaging using the dyes LD540, EGFP, DAPI and Alexa647
**Fig. 6****.** Photobleaching studies of 3 different dyes (LD540, Bodipy 493/503 and Nile Red) dissolved at 0.1 µg/ml in sunflower oil under constant illumination
**Fig. 7****.** Photobleaching studies of A431 cells stained with Bodipy 493/503 (0.5 µg/ml) or LD540 (0.1 µg/ml) under laser excitation

### EXAMPLE 1

### Synthesis of LD540

Synthesis was performed according to the general method given in U.S. Patent No. 5,446,157 with some minor modifications. Briefly, 1 g of tetrahydroindole was dissolved in 6 ml dry dichloromethane (DCM). Freshly distilled acetyl chloride (2 ml) was added dropwise. In an exothermic reaction, the solution turned dark red and was kept at 42 °C for 1 h. The solvent was evaporated and the residue triturated with 2 x 10 ml hexane. The residue was dissolved in 100 ml DCM and 2.5 ml triethylamine and 2.45 ml BF₃-etherate were added. After stirring for 2 h, the solvent was evaporated and the residue subjected to silica gel chromatography using DCM/hexane 2/3 as a solvent.

Yield: 350 mg of 4,4-Difluoro-2.3,5.6-bis-tetramethylene-4-bora-3a,4a-diaza-s-indacene (LD540, formula II), purity > 95%. The product was stored in sealed ampoules in aliquots of 5 mg at -20 °C.

UV-VIS spectroscopy: λₘₐₓ = 536 nm, ε = 89500 mol⁻¹cm⁻¹ (10 µM in ethanol) (Lit. (U.S. Patent No. 5,446,157): 535 nm, ε = 85100 mol⁻¹cm⁻¹)

### EXAMPLE 2

### Comparative lipophilicity studies of LD540 and Bodipy 493/503

Dyes were dissolved in octanol-saturated water or oil-saturated methanol at a concentration of 0.1 µg/ml, measured in the fluorometer and shaken with an equal volume of water-saturated octanol or methanol-saturated oil for 20 min. Phases were separated by centrifugation. For octanol/water coefficients, the concentration of dye in the water phase was measured and used for calculations of log P. For the oil/methanol system, 10 µl samples of both phases were taken, diluted into 990 µl of isopropanol and measured in the fluorometer. The ratio was used for calculation of log p. Values obtained in the octanol/water system indicated higher lipophilicity of LD540. Since log P values larger 3 obtained by direct solvent partitioning are prone to errors, we also measured in the immiscible pair sunflower oil/methanol, where log p values for lipophilic substances are much smaller and less error prone. Note that in both solvent pairs LD540 has significantly larger values of log P, indicating higher lipophilicity. All values are average +/- StdDev, p values were determined by Students t-test.

**Table 1**

| | log *P* LD540 | | log *P* Bodipy 493/503 | | Δlog *P* | |
|---|---|---|---|---|---|---|
| Octanol/water | 4.38 | +/- | 3.50 | +/- | 0.88 | +/- |
| | 0.27 | | 0.04 | | 0.32 | |
| | n = 7 | | n = 7 | | p = 0.0001 | |
| Oil/methanol | 0.75 | +/- | 0.31 | +/- | 0.44 | +/- |
| | 0.03 | | 0.02 | | 0.05 | |
| | n = 3 | | n = 3 | | p = 0.0002 | |

### EXAMPLE 3

### Measurement of spectral characteristics of LD540 under various conditions

**Table 2**

| | | |
|---|---|---|
| Solvent | Λ max exc | λ max em |
| Water | 534.8 | 542.4 |
| Ethanol^{#} | 536.0 | 542.5 |
| Hexane | 539.7 | 542.2 |
| Sunflower oil | 540.2 | 545.2 |
| 3T3-L1 cells | 540.2 | 545.0 |

Spectra were measured on a Jobin Yvon Fluoromax3 spectrofluorometer at 3 x 10⁻⁷ M. Differentiated 3T3-L1 cells were stained with 0.2 µg/ml LD540 in DMEM + 10% FCS for 30 min, followed by three washes. Cells were trypsinized, suspended at 100.000 cells/ml in PBS and measured in a fluorescence cuvette.

^{#}Note that the excitation maximum fits to the one reported in U.S. Patent No. 5,446,157, while the emission maximum in said reference was indicated to be 560 nm. At the concentration used there, 2 x 10⁻⁴ M, a maximum of 557 nm (in a 4 mm cuvette) was found, which decreased to 550 nm if a 1 mm cuvette was used, indicating that this red-shift is due to inner filtering at high concentration.

### EXAMPLE 4

### Comparative spectral anaysis of LD540 and other excitable fluorescent dyes

Initially, different dyes including LD540 were dissolved at 0.1 µg/ml in sunflower oil and both excitation and emission spectra measured on a Yobin-Yvon Fluoromax 3 spectrofluorometer. The results are shown in Fig. 5a. Note the narrow emission peak of LD540, which is the basis of its use for multi-color imaging.

An overlay of the LD540 spectra from Fig. 5a with the fluorescence spectra of EGFP (obtained from the Boswell&McNamara spectra collection at http://home.-earthlink.net/-gfpology/McNamara Boswell Spectra Dyes FPs.zi p). is shown in Fig. 5b. The position of laser lines and filter sets are indicated at the top of the picture.

The broad excitation spectrum of EGFP allows excitation at 458 nm (61% efficiency), where LD540 is only weakly activated (5% eff.). A BP475-515 nm emission filter collects a large part of EGFP fluorescence (45% of total emission) but a negligible amount of the LD540 emission. Likewise, excitation of LD540 at 543 (93% eff.) or 532 nm (68% eff.) together with a LP560 nm filter collects enough light (37% of total emission) for bright images.

The composite spectrum of Fig. 5c demonstrates a suitable arrangement of dyes including LD540 for 4-color imaging. Alongside with the spectra of the dyes used, the emission filter sets that were used for images in Fig. 3 are indicated at the top of the figure. Note the large gap between the emission of LD540 and Alexa647, which might be filled with another dye with excitation by the 594 nm laser line and narrow band emission around 610 nm, e.g. Texas Red.

### EXAMPLE 5

### Photobleaching studies

In a first experiment, the different dyes to be compared (LD540, Nile Red and Bodipy 493/503) were dissolved at 0.1 µg/ml in sunflower oil (which represents the cellular environment of the dyes) and constantly irradiated in a photometer cuvette at their respective absorption maximum. Excitation energy was equal for all three dyes. Every 15 seconds, fluorescence emission was recorded. The plot (Fig. 6) shows a logarithmic representation of the normalized fluorescence values. The slope of the curves are directly proportional to the respective rate of photobleaching. Under these conditions, LD540 bleaches 3-times slower than Nile Red and 15-times slower than Bodipy 493/503.

In a second experiment, A431 cells were stained with Bodipy 493/503 (0.5 µg/ml) or LD540 (0.1 µg/ml) as described above. On a Zeiss LSM510Meta, dyes were excited with a 488 nm or a 543 nm laser line, respectively, and imaging parameters were set for each dye individually to obtain optimal images. Repeated image frames were taken. On each series of frames, five regions of interest (ROIs), each containing several LDs, were defined. The pixel intensity values for each ROI were averaged to give the intensity that is plotted against the frame number. The resulting curve represents the bleaching rate that an experimentalist is practically faced to. Fig. 7 demonstrates that under these conditions, LD540 is about 4 times more stable than Bodipy 493/503, which corroborates the trend seen in Fig 6.

### EXAMPLE 6

### Staining of cells with LD540

**Fixed cells:** For a stock solution, LD540 was dissolved in ethanol at 0.5 mg/ml, which was stored at -20 °C. Cells were fixed with 4% paraformaldehyde, permeabilized with saponin and immuno-stained with antibodies as described previously (18). Cells were incubated with a solution of 0.05-0.1 µg/ml LD540 in PBS for 5-30 min, washed three times with PBS and once with water and mounted in Mowiol/Dabco as described (Kuerschner L, Moessinger C, Thiele C. Imaging of Lipid Biosynthesis: How a Neutral Lipid Enters Lipid Droplets. Traffic 2008; 9(3):338-352). When antibody staining was not necessary, cells were only fixed without permeabilization and stained with LD540 as described above.

**Living cells:** Normal growth medium was exchanged for DMEM w/o phenol red, 25 mM HEPES pH 7.4 (Gibco Nr. 21063) supplemented with 10% FCS (live cell imaging medium, LCIM). Cells were stained with LD540 at 0.5 µg/ml in LCIM for 10 min followed by 3 washes with LCIM. The higher concentration of LD540 needed is probably due to the presence of large amounts of BSA in the FCS, which captures some of the dye.

### EXAMPLE 7

### Imaging LD540 on confocal laser scanning microscopes

Implementation of a new dye on existing microscopes usually is a compromise between considerations of costs, time, flexibility and image quality, as will become obvious in the comments on the settings given below. Changing laser lines and scan head filters, which might be necessary to get optimal images, causes major costs, loss of time, and may compromise other applications. Therefore, microscope settings on two available Zeiss LSM510 instruments at the MPI-CBG light microscopy facility without any modifications of the instrument hardware were developed. The settings developed should enable transfer to many other microscopes, also of other manufacturers. In order to assure broad applicability, no use of spectral detection was made as well, although a suitable Zeiss LSM510 META detector and an Olympus Fluoview FV1000 system were available. Even with suboptimal settings, good images were obtained, supported by the dye's exceptional brightness and stability.

Fig. 1 shows formaldehyde-fixed cells as indicated which had been stained with a solution of 50 ng/ml LD540 in PBS for 10 min, washed with PBS and mounted in Mowiol/DABCO. Images were captured using a Zeiss LSM510Meta confocal microscope with laser excitation at 543 nm and emission filtering using a LP560 filter. Unprocessed original tiff files are shown. Note the very small but well-defined LDs in HuH7 cells and the virtual lack of any background from unspecific membrane staining. Bar: 10 µm.

Fig. 2 shows 2-color images of 3T3-L1 cells transiently expressing AUP1-EGFP. Cells were fixed, stained with LD540 and imaged as described above. An overlay of green (AUP1-EGFP) and red (LDs) channels is shown together with the single unprocessed tiff files to demonstrate the complete separation of the two channels.

Fig. 3 shows multi-color imaging of LDs in HuH7 hepatoma cells. HuH7 cells transiently expressing Rab18-EGFP (green) were fixed, permeabilized, and stained with Alexa647-phalloidin (magenta) for actin filaments, LD540 (red) for LDs and DAPI (blue) for DNA. Bar: 10 µm.

Fig. 4 shows COS7 cells transiently expressing tubulin-EGFP which were stained with LD540 and images had been taken on the Zeiss LSM510 META every 4 seconds. The images show LDs (magenta) and microtubules (green). The nucleus with the MTOC is to the upper left side of this image detail. Some LDs (yellow arrows) are stationary while others move either towards the nucleus (white arrow) or to the cell periphery (cyan arrows). Bar, 5 µm.

### Microscope settings for specific implementations:

In the following, the Zeiss nomenclature for designation of beam splitters and filters is used.

The main dichroic beamsplitters are designated **HFT XXX/YYY/...** (HFT stands for HauptFarbTeiler) with XXX etc. being the wavelengths of the light deflected onto the specimen. Emitted fluorescent light is allowed to pass through the HFT to the detector.

The secondary dichroic beamsplitters are designated **NFT XXX** (NFT stands for NebenFarbTeiler). Light with shorter wavelength than is deflected, light with longer wavelength passes the NFT.

An **LP XXX** (Long Pass) filter transmits emission light with wavelengths longer than the indicated threshold value XXX.

A **BP XXX-YYY** (Band Pass) transmits emission light within the indicated wavelength band.

### One-color imaging:

Any laser line between 488 and 543 nm can be used for excitation of LD540. When 488, 514, or 532 nm are used, the peak of emission can be collected with a suitable bandpass or longpass filter, depending on the necessity to reduce cellular background fluorescence. Excitation at 543 nm should be combined with a LP560 filter.

**2-color imaging** at the Zeiss LSM510 META:
**EGFP:** 458-nm line from a 40 mW Argon ion multi laser line at 14% laser power → HFT458/543 → sample → HFT458/543 → mirror → NFT515 → BP475-520 → detector 1
**LD540:** 543-nm laser from a 3 mW HeNe laser at 2% laser power → HFT458/543 → sample → HFT458/543 → mirror → NFT515 → Long pass filter LP560 → detector 2

The above settings are optimized for a bright EGFP signal, combined with optimal separation of channels. The same beam splitters are used for both channels, which allows arallel imaging of the two channels for fast frame acquisition, i.e. in living cells. Note that detector 1 effectively collects the EGFP emission between 475 and 515 nm because the NFT515 eliminates the light between 515 and 520 nm. This is important to exclude residual light emitted by LD540. LD540 is maximally excited at 543 nm with very low laser energy, resulting in extremely low photobleaching.

In general, these settings are close to optimal and result in excellent images. Potential for further improvement is in the use of a 532 nm laser line for excitation, which would allow to shift the emission filter for LD540 to slightly shorter wavelengths and collect even more light from the dye.

**3-color imaging** at the Zeiss LSM510 Meta:
**EGFP:** 458-nm line from a 40 mW Argon ion multi laser line at 14% laser power → HFT458/543 → sample → HFT458/543 → NFT635vis → NFT515 → BP475-520 → detector1
**LD540:** 543-nm laser from a 3 mW HeNe laser at 2% laser power → HFT458/543 → sample → HFT458/543 → NFT635vis → NFT515 → LP560 → detector2
**Alexa647:** 633-nm line from a 5 mW HeNe laser at 5% laser power → HFT514/633 → sample →HFT514/633 → NFT635vis Plate → META detector 640-800 nm

This implementation is based on the above 2-color settings. An additional NFT635vis secondary dichroic mirror splits the beam to separate the long wavelength emission. Since the 633 nm laser requires a different primary dichroic mirror, the third channel needs to be scanned separately, which precludes high frame rates. For the detection, the META detector was used, but only to substitute for a 640 nm long pass filter, which is lacking in the scan head. If nuclear staining is not required, as for most applications relevant for LD research, this is a reasonable well-optimized imaging setup. The far-red channel, now occupied with an Alexa 647 dye, may also be used with a red fluorescent protein. In this case, it may be appropriate to use a 594 nm laser line instead of the 633 nm line.

**4-color imaging** at the Zeiss LSM510:
**DAPI:** 405-nm laser from a 25 mW diode laser at 3% laser power → HFT405/514/633 → sample → HFT405/514/633 → NFT635vis → mirror → BP420-480 → detector 2
**EGFP:** 458-nm line from a 30 mW Argon ion multi laser line at 25% laser power → HFT458/514 → sample → HFT458/514 → NFT635vis → NFT515 → BP505-550 → detector 2
**LD540:** 514-nm line from a 30 mW Argon ion multi laser line at 6% laser power → HFT458/514 → sample → HFT458/514 → NFT635vis → NFT515 → BP530-580 → detector 3
**Alexa647**: 633-nm line from a 5 mW HeNe laser at 5% laser power → HFT405/514/633 → sample → HFT405/514/633 → mirror → NFT515 → LP650 → detector 3

This implementation was on a different instrument, because the LSM510 META is not equipped with a 405 nm laser line for a DNA dye. The instrument used, in contrast, does not feature a 543 nm laser, which required to excite LD540 off-center with the 514 nm laser line. This is compensated by the dyes brightness and by the use of a BP530-580 that collects most of the emitted light. The 514 nm laser line also weakly excites EGFP, whose fluorescence is partially collected with the BP530-580, potentially causing bleed-through into the LD540 image. Practically, this was not observed, probably because the dye is much brighter than EGFP. A more severe problem was encountered in the EGFP channel. The instrument is optimized for EGFP excitation with a 488 nm laser line and does not offer the BP475-520 but a BP505-550 emission filter. Combined with the NFT515 to remove possible fluorescence from LD540, this results in a narrow-band 505-515 nm collection of EGFP emission. This was partially compensated by increasing the excitation energy (25% vs. 14% before). Image quality was sufficient for well-expressed EGFP constructs, but low-expressing constructs or those with a diffuse intracellular distribution may result in noisy images. Although the instrument used was not optimal for this purpose, 4-color images of a quality sufficient for many practical uses were obtained.

For live cell imaging on the Zeiss LSM510 META, dishes were placed in a temperature controlled aluminum block. The objective was a Zeiss Plan-APOCHROMAT 63x/NA1.0 VIS/IR water immersion lens directly dipping into the cell culture dish, heated to 37°C.

Summarizing, the present invention provides the use of lipophilic dyes derived from the bodipy fluorophore, LD540, for microscopic imaging of lipophilic cellular components, in particular lipid droplets. In contrast to previous lipid droplet dyes, LD540 and structurally related compounds can spectrally be resolved from both green and red fluorophores allowing multi-color imaging in both fixed and living cells. Their improved staining specificity, brightness and photostability relative to commonly used dyes such as Bodipy 493/503 and Nile Red supports live cell imaging, which was used to demonstrate by two-color imaging lipid droplet motility along microtubules. The improved staining specificity, brightness and photostability of these dyes of formula I is also advantageous in various other applications such as flow cytometry and FACS.

## Claims

1. Use of a bodipy dye having the following structural formula I 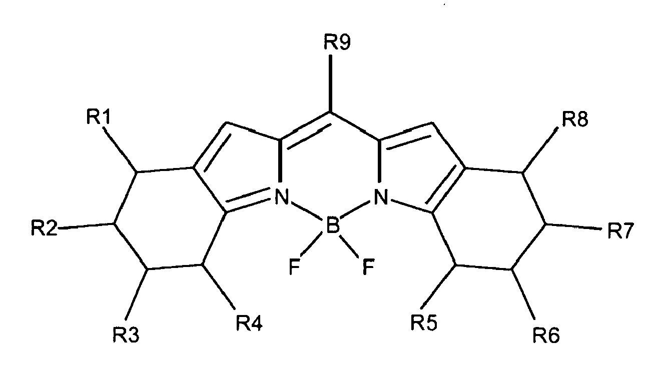 wherein the substituents R₁, R₂, R₃, R₄, R₅, R₆, R₇, R₈, and R₉ independently are H or an organic residue, in particular an unsubstituted or substituted (cyclo)alkyl group, alkenyl group, alkinyl group, heterocyloalkyl group, aryl group, heteroaryl group, a functional group comprising at least one heteroatom such as N, O, S, P or halogen, or any combination of said groups,
for fluorescence-based imaging and/or analysis of cells and/or cellular components.

2. The use according to claim 1, wherein R₁, R₂, R₃, R₄, R₅, R₆, R₇, R₈ and R₉ independently are H or an unsubstituted or substituted lower alkyl group having 1-8 C atoms.

3. The use according to claim 2, wherein R₁, R₂, R₃, R₄, R₅, R₆, R₇, R₈ are each H and R₉ is an unsubstituted or substituted lower alkyl group having 1-8 C atoms.

4. The use according to claim 3, wherein R₉ is methyl, ethyl, propyl or butyl.

5. The use according to any one of claims 1-4, wherein living cells are subjected to said fluorescence-based imaging or analysis of cells and/or cellular components.

6. The use according to any one of claims 1-5, wherein the cellular components comprise lipid droplets which are stained with the bodipy dye as defined in any one of claims 1-4.

7. The use according to any one of claims 1-6, wherein said fluorescence-based imaging or analysis is a multi-color imaging or analysis.

8. A method for fluorescence-based imaging or analysis of cells and/or cellular components, comprising the following steps:
a) staining cellular components with a bodipy dye of formula I as defined in any one of claims 1-4;
b) staining other cellular components with at least one other excitable fluorescent dye having a different maximum of the adsorption and/or emission spectrum than the bodipy dye used in step a);
c) exciting the bodipy dye of step a) with laser light in the wave length range from 450 nm to 565 nm;
d) exciting the at least one other excitable fluorescent dye of step b) with laser light in a wave length range different from that used to excite the bodipy dye of formula I;
e) detecting the light emitted by the different dyes used in step a) and step b);
f) optionally generating images with the emission data obtained in step e);
g) optionally performing an analysis with the data obtained in step e) or the images obtained in step f).

9. The method according to claim 8, wherein the cellular components stained with the bodipy dye of formula I in step a) are lipid droplets.

10. The method according to claim 8 or 9, wherein the at least one other excitable fluorescent dye used in step b) is selected from the group comprising Cy dyes, Alexa dyes such as Alexa 647, NBD, fluorescein and its derivatives such as cascade blue and Oregon green, fluorescent proteins such as EGFP, emerald, EYFP, mRFP, morange, mPlum, mcherry, DAPI, and Texas Red.

11. The method according to any one of claims 8-10, wherein the wave length of the laser light used for exciting the bodipye dye in step a) is 488 nm, 514 nm, 532 nm or 543 nm.

12. The method according to any one of claims 8-11, wherein 1, 2 or 3 other excitable fluorescent dye(s) are used in step b), resulting in 2-color, 3-color or 4-color imaging.

13. The method according to anyone of claims 8-12, wherein living cells are subjected to said fluorescence-based imaging or analysis of cells and/or cellular components.

14. The method according to any one of claims 8-13, wherein a confocal laser scanning microscope, a spinning disc microscope, a two-photon microscope, a conventional epifluorescence microscope, a structured illumination microscope, or a single plane illumination microscope is used.

15. The use according to any one of claims 1-7 or the method according to any one of claims 8-14 which involves microscopy, flow cytometry or fluorescence activated cell sorting (FACS).
